# EUROPEAN PATENT APPLICATION

(11) **EP 1 403 364 A1**
(43) Date of publication of application: **31.03.2004**
(21) Application number: 02733398.8
(22) Date of filing: 07.06.2002
(51) Int. Cl.: C12N 9/12, C12N 15/09

(54) **MUTANT RNA POLYMERASES**

(30) Priority: 11.06.2001 JP 2001175886; 30.04.2002 JP 2002128688
(71) Applicant: Nippon Genetech Co. Ltd., Tokyo 101-0054 (JP)
(72) Inventor: WATAHIKI, Masanori, c/o Toyama Laboratory, Toyama-shi, Toyama 930-0982 (JP); YONEDA, Yuko, c/o Toyama Laboratory, Toyama-shi, Toyama 930-0982 (JP)
(74) Representative: VOSSIUS & PARTNER
(86) International application number: PCT/JP2002/005670
(87) International publication number: WO 2002/101032

(57) **Abstract**

It is intended to provide novel mutant RNA polymerases enabling a transcriptional sequencing method whereby a high SN ratio can be achieved in sequence analysis with the use of capillary and longer and more accurate base sequencial data can be obtained by a single reaction. More specifically, a mutant RNA polymerase derived from a wild type RNA polymerase by substitution of at least one amino acid and deletion of at least one amino acid, or a mutant RNA polymerase derived from a wild type RNA polymerase by deletion of at least one amino acid, wherein the above-described substitution and/or deletion of amino acid(s) have been performed so that the resultant mutant RNA polymerase has an enhanced ability to incorporate 3'-deoxynucleotide as a substrate compared with the wild type RNA polymerase corresponding thereto.

## Description

### Technical Field

The present invention relates to mutant RNA polymerase that is useful in base sequencing methods employing transcription reactions.

### Background Art

DNA sequencing methods are essential methods of obtaining information about the sequence of the genome of an organism. Historically, base sequencing methods have consisted of the chemical degradation method (Maxam, A.M., et al., Proc. Natl. Acad. Sci., 74: 1258-1262 (1977)) and enzyme synthesis method (Sanger, F., et al., Proc. Natl. Acad. Sci. USA, 74: 5463 (1977)). The former is known as the Maxam-Gilbert method and the latter as the dideoxy terminator method, or Sanger method, after its developer.

Currently, the cycle sequencing method employing DNA polymerase, particularly thermostable DNA polymerase, based on the Sanger method, is widely employed as the general method of sequencing bases. The series of operations used to identify bases in the cycle sequencing method comprises: (1) preparation of a template, (2) preparation of DNA fragments labeled by a partial termination reaction employing DNA polymerase, (3) DNA fragment electrophoresis, (4) the reading of DNA fragments that have been subjected to electrophoresis, and (5) analyzing the results that have been read.

With regard to steps (2) through (5), an automatic DNA sequencer has been developed which employs four dideoxy nucleotides each labeled with one of four different fluorescent dyes having different wavelength characteristics. While conducting electrophoresis, the specific excitation wavelength of the fluorescent dye employed is detected by irradiation with a laser to automatically and sequentially read the lengths of the DNA fragments, which are then converted to sequence data. This makes it possible to readily obtain large amounts of DNA sequence information (Smith, L.M., et al., Nature, 321: 674 (1986)).

However, the preparation in (1) of a template suited to the polymerase reaction of (2) is important. Since this preparation reflects the quantity and quality of data obtained in (4), a variety of efforts have been made. To simplify the operation, the current practice is to use the method of employing templates in the form of amplification products obtained by a gene amplification method (PCR) employing thermostable DNA polymerase (Phear, G.A., et al., Methods Mol. Biol., 31: 247 (1994): Shigeru TAKAKI, Proteins, Nucleic Acids, and Enzymes, 37(5): 868 (1992)). However, although the preparation of templates is extremely easy in this method, unreacted primer and unincorporated deoxy nucleotides picked up from the previous PCR reaction block the sequencing reaction employing DNA polymerase, often precluding sequence analysis. Accordingly, to solve this problem, methods of purifying the PCR product and enzymatically inactivating the primer and free nucleotides have been devised: However, both methods involve tedious operations and are not suited to large-scale analysis due to their cost.

The transcriptional sequencing method, permitting the direct use of the PCR product without further purification, has been developed as a solution to the problems encountered in methods employing PCR amplified product as template (Sasaki, N., et al., Proc. Natl. Acad. Sci. USA, 95(7): 3455 (1998); Japanese Patent No. 3,155,279; and Japanese Unexamined Patent Publication (KOKAI) Heisei No. 11-75898). This method permits omission of the tedious operation step of purifying the PCR reaction product and can be expected to contribute to a reduction in overall cost.

The transcriptional sequencing method employs phage RNA polymerase and is based on the principles of conducting a transcription reaction in the presence of 3'-deoxy nucleotide or a derivative thereof, separating RNA fragments by electrophoresis, and reading the base sequence based on the size of the RNA fragments. When a mutant of T7 RNA polymerase (Japanese Unexamined Patent Publication (KOKAI) Heisei No. 11-75867) such as the T7 RNA polymerase F644Y is employed as the RNA polymerase, incorporation is possible without the bias of 3'-deoxy nucleotide or a derivative thereof, permitting more accurate base sequencing (Unexamined Japanese Patent Publication (KOKAI) Heisei No. 11-75898).

However, in the transcriptional sequencing method, as well, there are problems in that noise is high and correct sequencing analysis is sometimes impossible. This noise problem is not encountered with conventional slab sequencers. This is thought to be due to the relatively large cross-sectional area as a sample moves through a substrate of polyacrylamide. However, noise is high in sequencers employing capillaries, sometimes precluding correct sequence analysis. Since the cross-sectional area of a capillary is quite small, there is a limit to the size of the sample load that can be withstood. Thus, device sensitivity is high and there is thought to be extremely high susceptibility to noise. Since it is possible to analyze a large sequence at once in a sequencer employing capillaries, such sequencers are necessary for the processing of large amounts of sample processing. Accordingly, it is urgent to solve the above-stated problems in order for transcriptional sequencing to become more widespread.

There is also a need for improvement in methods capable of obtaining more precise base sequence data for longer DNA chains in a single polymerase reaction.

A summary edition of the human genome has been published (Venter, J.C., et al., Science, 291:1304-1351 (2001); International Human Genome Sequencing Consortium, Nature, 409: 860-921 (2001)). The human genome sequence is said to comprise 3 billion base pairs. Using current base sequencing methods, in a single reaction, it is possible to determine about 500 bases, and even when effort is expended to read even longer sequences, the number of base pairs that can be analyzed is about 1000. Accordingly, it is necessary to conduct an extremely large number of reactions to determine the base sequence of such an immense genome. Accordingly, a technique for reading long strands of DNA in a single polymerase reaction is necessary.

Further, fragment information about base sequences determined by the above-mentioned technique is assembled into the continuous base sequence information of the genome of an organism. The field of bioinformatics is a rapidly developing field that compares sequence information obtained from numerous organisms, anticipates functional analysis from the sequence information, and links the information to disease gene identification, drug creation, and gene diagnosis (for example, Genetic Medicine, Vol. 4, Issue 3 (2000)). Recently, in particular, single nucleotide polymorphism (SNP) has garnered attention; this appears to explain phenomena specifying personal differences and the variety found in life. A large amount of research attempting to apply this information to susceptibility to disease, drug sensitivity, drug resistance, side effects, and other treatment taking personal differences into consideration is being conducted. Thus, the task of base sequencing is still thought to be quite large. In particular, in the analysis of SNPs, it is necessary to more accurately obtain base sequence data.

Progress in base sequencing methods has permitted analysis on the scale of the human genome. In the future, it is thought that analysis of individual genetic information and analysis of the genomes of organisms other than humans as part of gene function analysis will produce increasing demand for base sequencing techniques. It is thought that providing an improved method of transcriptional sequencing having the above-described advantages will facilitate and permit more accurate base sequencing of larger numbers of bases, and will contribute greatly to future development of bioinformatics.

Accordingly, the object of the present invention is to provide a new mutant RNA polymerase yielding a high S/N ratio even in capillary sequence analysis, permitting a transcriptional sequence method yielding more accurate and longer base sequence data in a single reaction.

One major characteristic of the transcriptional sequencing method is the use of RNA polymerase derived from phages known as T3 and T7 that infect *Escherichia coli* (*E. coli*), SP6 phage infecting *Salmonella* bacteria, or K11 phage infecting *Klebsiella pneumoniae* that are highly specific to promoter sequences. RNA fractions are synthesized in *in vitro* transcription reactions to determine base sequences.

Generally, in the series of transcription reactions occurring within an organism, at the end of transcription, newly produced RNA molecules and RNA polymerase are released. The RNA polymerase that is released is known to recognize another promoter and enter the transcription stage. In this process, highly active T7 RNA polymerase transcribes several hundred copies of an RNA molecule from a single template under optimum conditions, having the ability to produce new RNA molecules. This indicates that T7 RNA polymerase, like other RNA polymerases, has the function of terminating transcription. Further, transcription termination phenomena differing from that occurring *in vivo* are known to occur based on the structure of the template employed when T7 RNA transcriptase is used to conduct transcription reactions *in vitro* (Schenborn, E.T., et al., Nuc. Acids Res., 13(17): 6223 (1985)).

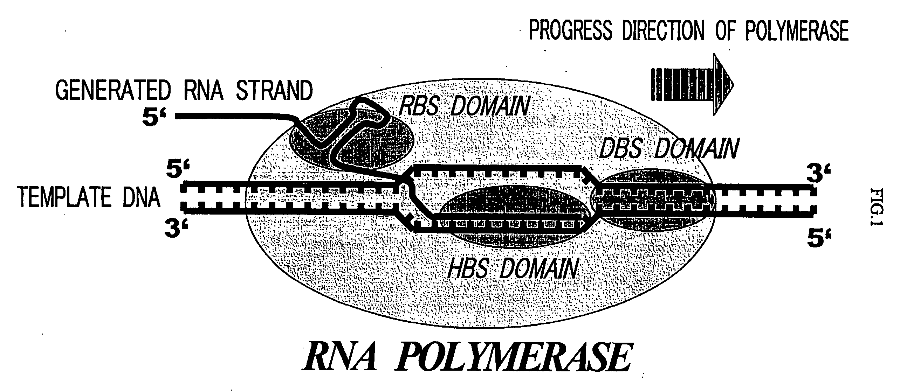

The transcription extension reaction (transcription extension reaction complex) will be described first based on the schematic diagram of Fig. 1.

It is known that in the structural portion of the protein of RNA polymerase, a double-strand binding domain (DBS domain), a portion which encircles the double helix of DNA serving a template, a portion recognizing hybrid of template DNA and RNA in the process of synthesis (HBS domain; hybrid binding domain) and a portion binding with just RNA strands in the form of RNA separated from the hybrid (RBS domain; RNA binding domain). During the formation of a transcription extension reaction complex, transcription is terminated when these three portions reach the sequence in the template DNA prompting the end of transcription. In particular, when new RNA binding to the RBS domain forms a stem loop structure and, for example, a sequence of consecutive Us is present in the RNA strand in a DNA-RNA hybrid present in the HBS domain present downstream therefrom, the RNA that has been synthesized is effectively released from the template. This free RNA is thought to code for essential proteins (Jeng, S. T., et al., J. Biol. Chem., 265(7): 3823 (1990)).

However, when substrate without a group binding to the next nucleotide, such as a 3'-deoxy nucleotide, is mixed into some of the substrate, as occurs in the transcriptional sequencing method, the nucleotide immediately following incorporation of 3'-deoxy nucleotide is not incorporated into the RNA strand, effectively forcing transcription to terminate. Since the transcription extension reaction is forced to stop in this case, differing from the above-described mechanism, many RNA polymerases are known to be unable to separate from the template, forming complexes in which the RNA polymerase remains in the template. (Tyagarajan, K., et al., Biochemistry, 30(45): 10920 (1991)). However, in the transcriptional sequencing, sequence analysis is possible even when transcription is stopped by 3'-deoxy nucleotides and a complex is formed with the RNA polymerase still bonded. This is thought to occur because certain amount of RNA polymerase is thought to be released from the RNA in the process of purifying the transcription product by gel filtration or ethanol precipitation and in electrophoresis in a modified gel containing a high concentration of urea, so that the formation of the above complex does not affect transcriptional sequencing (Sasaki, N., et al., Proc. Natl. Acad. Sci. USA, 95(7): 3455 (1998)).

However, as stated above, the problem of noise has been pointed out in sequence analysis employing capillaries that requires highly sensitive detection. The fact that there is some relation between this high noise problem and the formation of the above-described complex and the fact that there is some relation between the formation of the complex and the possibility of synthesizing long strands of RNA and the accuracy of base sequence analysis have never before been pointed out.

The present inventors received a hint in the form of reports that the transcription termination anomalies observed in wild T7 RNA polymerase were not observed in T7 RNA polymerase having a certain mutation, and conceived that it might be possible to solve the above-stated problem of high noise, conducting a broad investigation. The present invention was devised on that basis. More specifically, the present inventors discovered that, for example, mutant T7 RNA polymerase DEL 172-173 and mutant T7 RNA polymerase F644Y/DEL 172-173, obtained by adding a deficiency such as in the amino acids Lys-172 and Arg-173 to wild T7 RNA polymerase and mutant T7 RNA polymerase F644Y, yielded improved S/N ratio results in transcriptional sequencing. The present inventors further discovered that these mutants deficient in the amino acids Lys-172 and Arg-173 promoted the incorporation of 3'-deoxy nucleotides and their derivatives, confirming that more accurate base sequence analysis was possible and leading to the present invention.

### Disclosure of the Invention

That is, the present invention relates to mutant RNA polymerase (the first aspect of mutant RNA polymerase) in which at least one of the amino acids of wild RNA polymerase has been substituted and in which at least one amino acid has been rendered deficient, characterized in that the substitution and deficiency of said amino acids is conducted in such a manner as to enhance the ability to incorporate 3'-deoxy nucleotide as substrate relative to that of the corresponding wild RNA polymerase.

The present invention further relates to mutant RNA polymerase for use in transcriptional sequencing (the second aspect of the mutant RNA polymerase) in which at least one of the amino acids of wild RNA polymerase has been rendered deficient, characterized in that the deficiency of said amino acid is conducted in such a manner as to enhance the ability of incorporating 3'-deoxy nucleotide as substrate relative to that of the corresponding wild RNA polymerase.

### Brief Description of the Drawings

Fig. 1 is a schematic diagram showing a transcription extension reaction (a transcription extension reaction complex) with RNA polymerase (see the text for DBS, HBS, and RBS domains).

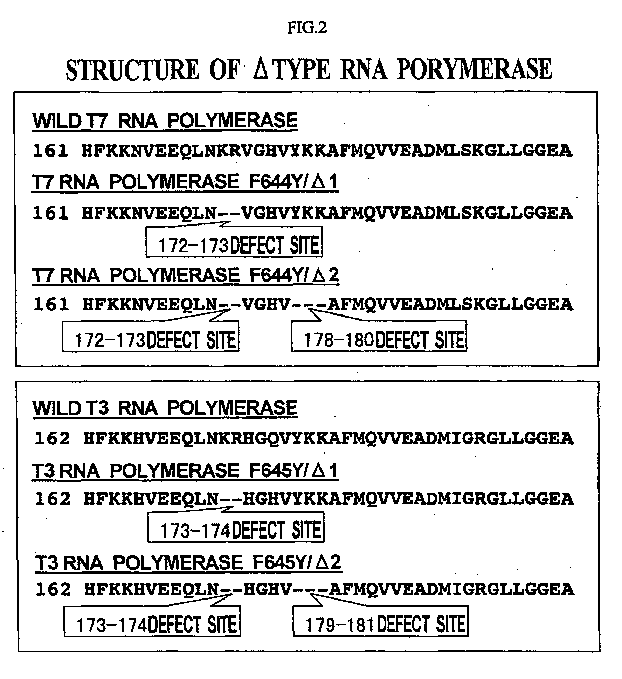

Fig. 2 is the amino acid sequence of the deficient region of deficient mutant enzyme in the form of T7 and T3 RNA polymerase (the number 161 in the T7 RNA polymerase and the number 162 in the T3RNA polymerase in the figure indicate the amino acid number (counting out from the N terminal of each enzyme) of the initial H (histidine) in the amino acid sequence of the polymerase shown; the sequences of T7 RNA polymerase and T3 RNA polymerase were extracted from data in DDBJ Accession Nos. V01146 and X02981).

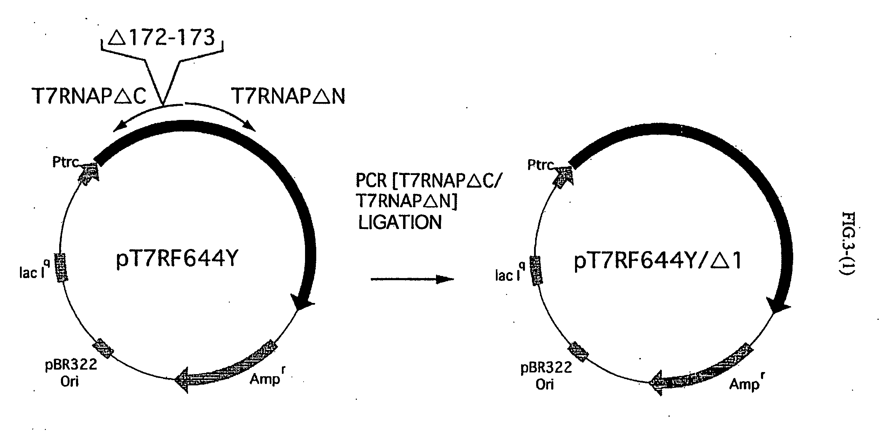

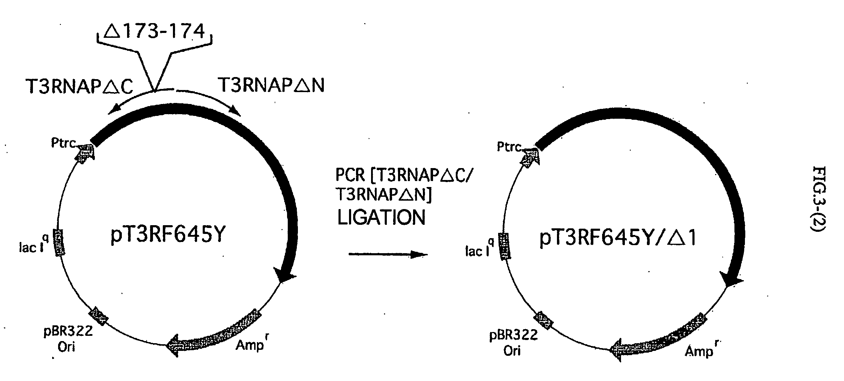

Fig. 3 is a schematic diagram of the method of preparing expression plasmid producing deficient mutant T7 RNA and T3RNA polymerases.

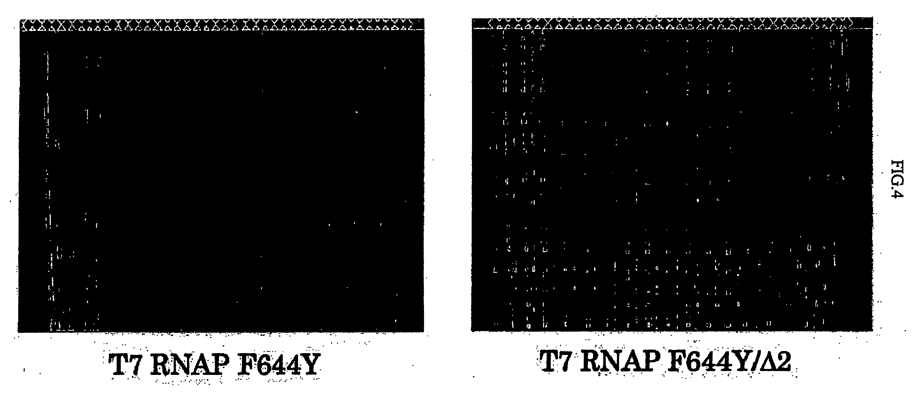

Fig. 4 is a comparison of the results of transcriptional sequencing conducted with mutant T7 RNA polymerase F644Y and deficient mutant T7 RNA polymerase F644Y/Δ2 (electrophoresis was conducted with an ABI 377 XL sequencer and the gel images were compared; the front end in electrophoresis is shown toward the bottom).

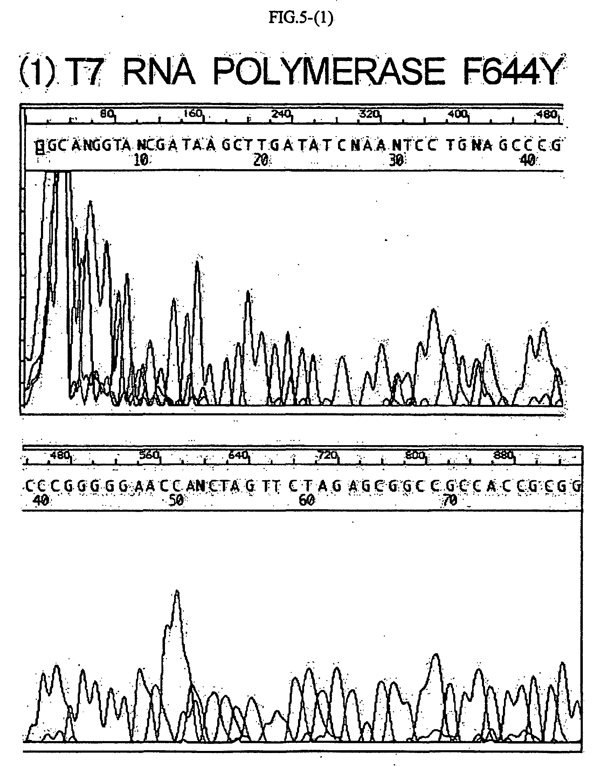

Fig. 5 is comparisons of the results of transcriptional sequencing conducted with mutant T7 RNA polymerase F644Y and deficient mutant T7 RNA polymerase F644Y/Δ2 (electrophoresis was conducted with an ABI 377 XL sequencer, base calls were made, and portions thereof were removed and compared; the results of Fig. 4 were employed).

### Best Mode of Implementing the Invention

### (Mutant RNA Polymerase)

The mutant RNA polymerase of the first aspect of the present invention is mutant RNA polymerase in which at least one of the amino acids of wild RNA polymerase has been substituted and in which at least one amino acid has been rendered deficient, characterized in that the substitution and deficiency of said amino acids is conducted in such a manner as to enhance the ability of incorporating 3'-deoxy nucleotide as substrate relative to that of the corresponding wild RNA polymerase..

The mutant RNA polymerase of the second aspect of the present invention is mutant RNA polymerase in which at least one of the amino acids of wild RNA polymerase has been rendered deficient, characterized in that the deficiency of said amino acid is conducted in such a manner as to enhance the ability of incorporating 3'-deoxy nucleotide as substrate relative to that of the corresponding wild RNA polymerase.

The 3'-deoxy nucleotide may be either 3'-deoxynucleotide having a label such as a fluorescent label or 3'-deoxy nucleotide not having a label.

The wild RNA polymerase serving as the base of the mutant RNA polymerase may be RNA polymerase derived from, for example, T7 phage, T3 phage, SP6 phage, or K11 phage.

Japanese Unexamined Patent Publication (KOKAI) Heisei No. 11-75867 (Japanese Patent No. 3,172,710) describes mutant RNA polymerase in which at least one of the amino acids of wild RNA polymerase has been substituted so as to enhance the ability to incorporate 3'-dNTP derivatives relative to the ability of the corresponding wild RNA polymerase.

Specifically, the mutant RNA polymerase described in the above-cited publication is derived from T7 phage, T3 phage, SP6 phage, or K11 phage. In the case of RNA polymerase derived from T7 phage, amino acid residue 644 or 667 is substituted with tyrosine. In the case of RNA polymerase derived from T3 phage, amino acid residue 645 or 688 is substituted with tyrosine. In the case of SP6 phage, amino acid residue 670 is substituted with tyrosine. And in the case of K11 phage, amino acid residue 690 is substituted with tyrosine.

By contrast, the mutant RNA polymerase of the first aspect of the present invention is RNA polymerase having an amino acid deficiency in addition to the above-described substitution, in which the ability to incorporate 3'-dNTP derivatives is enhanced relative to that of mutant RNA polymerase having just the above-described substitution.

The deficiency of an amino acid in the mutant RNA polymerase of the first and second aspects of the present invention is desirably effected in the region containing the basic amino acid present in the region upon which the protease of *E. coli* acts in the RNA polymerase protein. Here, "the region upon which the protease of *E. coli* acts" is beyond the Lys-172 or Lys-179 (depending on the strain of *E*. *coli*, this is sometimes beyond the Tyr-178) recognized by the protease cutting the T7 RNA polymerase protein into two peptides of 20 KDa and 80 KDa. In the case of trypsin protease (trypsin is an endopeptidase having affinity for the basic amino acids at the center of peptides and selectively cutting the C-terminal peptide bonds of Lys and Arg) causing degradation of the same T7 RNA polymerase protein, this region follows Lys-173 and Lys-180 (Daniel K. Muller, et al. Biochemistry, 27: 5763-5771 (1988)).

The amino acid deficiency region of the RNA polymerase in the present invention can be either the region containing a basic amino acid of Lys-172 and Arg-173 (Ikeda, et al. J. Biol. Chem., 262: 3190 (1987)) or Tyr-178, Lys-179, and Lys-180 (Grodberg, J. et al. J. Bacteriol., 170: 1245 (1988)) in T7 RNA polymerase. Similarly, in RNA polymerase derived from T3 phage, the amino acid deficiency region of the RNA polymerase can be any of the regions containing a basic amino acid from among amino acid residues 173, 174, 179, 180, and 181. In the case of K11 phage, it can be any region containing a basic amino acid from among residues 192, 193, 198, 199, and 200. In the case of RNA polymerase derived from SP6 phage, it can be any region containing a basic amino acid from among amino acid residues 136, 137, 140, 141, 142, and 143.

Further, the number of amino acids rendered deficient, especially the number of amino acids rendered deficient in the range containing a basic amino acid can be a continuous or intermittent sequence of from 1 to 10 amino acids comprising the above-stated amino acid residues. The number of amino acids in the continuous or intermittent amino acid sequence can be from 1 to 7 or from 1 to 5. Further, the number of amino acids rendered deficient in the region containing a basic amino acid can be 1, 2, or 3.

Nicked RNA polymerase, which is a segment of two peptides of 20 KDa and 80 · KDa produced by the decomposition of T7 RNA polymerase in the vicinity of amino acid residues 170-190, is known (Macdonald, L.E., et al. J. Mol. Biol., 232: 1030-1047 (1993)). Further, Lyakhov et al. report that deficient mutant T7 RNA polymerase known as Del 172-173 in which the two amino acid residues of Lys-172 and Arg-173 have been rendered deficient, in the same manner as the above nicked T7 RNA polymerase, is not stopped by a transcription stop signal known as the PTH signal; that T7 RNA polymerase Del 172-173 suppresses the synthesis of abnormal transcription products seen in wild RNA polymerase; and that the amino acid sequence in the vicinity of Lys-172 of T7 RNA polymerase exhibiting protease sensitivity is the same in similar polymerases such as T3, K11, and SP6 (Lyakhov, D.L., et al. Mol. Biol. 25(5):679-687 (1992)).

T7 RNA polymerase Del 172-173 is known to have the above-stated properties. However, its use in transcriptional sequencing and the results obtained thereby were not previously known. The report by Lyakhov et al. states only that T7 RNA polymerase Del 172-173 is suited to the production of RNA probes because it does not produce abnormal transcription products.

The present inventors discovered that by introducing a deficiency (Del 172-173) into amino acids 172 and 173 in the mutant T7 RNA polymerase described in the above-cited patent publication yielded mutant RNA polymerase with an increased ability to introduce 3'-deoxy nucleotide as substrate relative to the corresponding wild RNA polymerase. They further discovered that not just mutant RNA polymerase deficient in the amino acid 172 lysine and 173 arginine of the above-described T7 RNA polymerase, but also mutant RNA polymerase deficient in 178 tyrosine, 179 lysine, and 180 lysine, was mutant RNA polymerase with an enhanced ability to incorporate 3'-deoxy nucleotide as substrate relative to the corresponding wild RNA polymerase.

In addition, the present inventors discovered that when just a deficiency (Del 172-173) of amino acids 172 and 173 was introduced into wild T7 RNA polymerase, an enhanced ability to incorporate 3'-deoxynucleotide as substrate was exhibited relative to the corresponding wild RNA polymerase. Further, not just mutant RNA polymerase rendered deficient in the 172 lysine and 173 arginine amino acids of wild T7 RNA polymerase, but also mutant RNA polymerase rendered deficient in the 178 tyrosine, 179 lysine, and 180 lysine exhibited an enhanced ability to incorporate 3'-deoxy nucleotides as substrate relative to the corresponding wild RNA polymerase.

The mutant RNA polymerase of the first aspect of the present invention can be, for example, RNA polymerase obtained by substituting tyrosine for the phenylalanine at amino acid residue 644 or 667, or both 664 and 667, of RNA polymerase derived from T7 phage, and deleting the 172 lysine and/or 173 arginine amino acid residue.

Further, the mutant RNA polymerase of the first aspect of the present invention can be RNA polymerase obtained by substituting tyrosine for the phenylalanine at amino acid residue 644 or 667, or both 664 and 667, of RNA polymerase derived from T7 phage, and deleting at least one of the amino acid residues from among the three amino acid residues 178 through 180.

Further, the mutant RNA polymerase of the first aspect of the present invention can be RNA polymerase obtained by substituting tyrosine for the phenylalanine at amino acid residue 644 or 667, or both 664 and 667, of RNA polymerase derived from T7 phage; deleting the 172 lysine and/or 173 arginine amino acid residue; and deleting at least one of the amino acid residues from among the three amino acid residues 178 through 180.

Mutant RNA polymerase in which tyrosine has been substituted for the phenylalanine at amino acid residue 664 or 667, or both 664 and 667, of RNA polymerase derived from T7 phage is described in Japanese Unexamined Patent Publication (KOKAI) Heisei No. 11-75867 (Japanese Patent No. 3,172,710). In the mutant RNA polymerase employed in the present invention, a specific deficiency is further introduced into mutant RNA polymerase in which the above-stated phenylalanine has been replaced with tyrosine.

The mutant RNA polymerase of the first aspect of the present invention can be, for example, RNA polymerase obtained by substituting tyrosine for the phenylalanine at amino acid residue 645 or 668, or both 645 and 668, of RNA polymerase derived from T3 phage, and deleting the 173 lysine and/or 174 arginine amino acid residue.

Further, the mutant polymerase of the first aspect of the present invention can be RNA polymerase obtained by substituting tyrosine for the phenylalanine at amino acid residue 645 or 668, or 645 and 668, of RNA polymerase derived from T3 phage, and deleting at least one of the amino acid residues from among the three amino acid residues 179 through 181.

Still further, the mutant polymerase of the first aspect of the present invention can be RNA polymerase obtained by substituting tyrosine for the phenylalanine at amino acid residue 645 or 668, or 645 and 668, of RNA polymerase derived from T3 phage; deleting the 173 lysine and/or 174 arginine amino acid residue; and deleting at least one of the amino acid residues from among the three amino acid residues 179 through 181.

Mutant RNA polymerase in which tyrosine has been substituted for the phenylalanine at amino acid residue 645 or 668, or both 645 and 668, of RNA polymerase derived from T3 phage is described in Japanese Unexamined Patent Publication (KOKAI) Heisei No. 11-75867 (Japanese Patent No. 3,172,7 10). In the mutant RNA polymerase employed in the present invention, a specific deficiency is further introduced into mutant RNA polymerase in which the above-stated phenylalanine has been replaced with tyrosine.

The mutant polymerase of the first aspect of the present invention can be RNA polymerase obtained by substituting tyrosine for the phenylalanine at amino acid residue 690 of RNA polymerase derived from K11 phage and deleting the 192 lysine and/or 193 arginine amino acid residue.

Mutant RNA polymerase in which tyrosine has been substituted for the phenylalanine at amino acid residue 690 of RNA polymerase derived from K11 phage is described in Japanese Unexamined Patent Publication (KOKAI) Heisei No. 11-75867 (Japanese Patent No. 3,172,710). In the mutant RNA polymerase employed in the present invention, a specific deficiency is further introduced into mutant RNA polymerase in which the above-stated phenylalanine has been replaced with tyrosine.

Further, the mutant polymerase of the first aspect of the present invention can be RNA polymerase obtained by substituting tyrosine for the phenylalanine at amino acid residue 690 of RNA polymerase derived from K11 phage, and deleting at least one of the amino acid residues from among the three amino acid residues 198 through 200.

Still further, the mutant polymerase of the first aspect of the present invention can be RNA polymerase obtained by substituting tyrosine for the phenylalanine at amino acid residue 690 of RNA polymerase derived from K11 phage; deleting the 192 lysine and/or 193 arginine amino acid residue; and deleting at least one of the amino acid residues from among the three amino acid residues 198 through 200.

The mutant polymerase of the first aspect of the present invention can be, for example, RNA polymerase obtained by substituting tyrosine for the phenylalanine at amino acid residue 670 of RNA polymerase derived from SP6 phage, and deleting the lysine 136 and/or arginine 137 amino acid residue.

Mutant RNA polymerase in which tyrosine has been substituted for the phenylalanine at amino acid residue 670 of RNA polymerase derived from SP6 phage is described in Japanese Unexamined Patent Publication (KOKAI) Heisei No. 11-75867 (Japanese Patent No. 3,172,710). In the mutant RNA polymerase employed in the present invention, a specific deficiency is further introduced into mutant RNA polymerase in which the above-stated phenylalanine has been replaced with tyrosine.

The mutant polymerase of the first aspect of the present invention can be RNA polymerase obtained by substituting tyrosine for the phenylalanine at amino acid residue 670 of RNA polymerase derived from SP6 phage, and deleting at least one of the basic amino acids among the four amino acid residues 140 through 143.

Further, the mutant polymerase of the first aspect of the present invention can be RNA polymerase obtained by substituting tyrosine for the phenylalanine at amino acid residue 670 of RNA polymerase derived from SP6 phage; deleting the lysine 136 and/or arginine 137 amino acid residue; and deleting at least one of the basic amino acids among the four amino acid residues 140 through 143.

The mutant RNA polymerase of the second aspect of the present invention can be, for example, RNA polymerase rendered deficient in the 172 lysine and/or 173 arginine amino acid residues of RNA polymerase derived from T7 phage.

Further, the mutant RNA polymerase of the second aspect of the present invention can be RNA polymerase derived from T7 phage that has been rendered deficient in at least one of the three amino acid residues 178 through 180.

Still further, the mutant RNA polymerase of the second aspect of the present invention can be RNA polymerase rendered deficient in the 172 lysine and/or 173 arginine amino acid residues of RNA polymerase derived from T7 phage; and rendered deficient in at least one of the three amino acid residues 178 through 180.

The mutant RNA polymerase of the second aspect of the present invention can be, for example, RNA polymerase derived from T3 phage that has been rendered deficient in the 173 lysine and/or 174 arginine amino acid residues of RNA polymerase.

Further, the mutant RNA polymerase of the second aspect of the present invention can be RNA polymerase derived from T3 phage that has been rendered deficient in at least one of the three amino acid residues 179 through 181.

Still further, the mutant RNA polymerase of the second aspect of the present invention can be RNA polymerase derived from T3 phage that has been rendered deficient in the 173 lysine and/or 174 arginine amino acid residue; and rendered deficient in at least one of the three amino acid residues 179 through 181.

The mutant RNA polymerase of the second aspect of the present invention can be, for example, RNA polymerase derived from K11 phage that has been rendered deficient in the 192 lysine and/or 193 arginine amino acid residues of RNA polymerase.

Further, the mutant RNA polymerase of the second aspect of the present invention can be RNA polymerase derived from K11 phage that has been rendered deficient in at least one of the three amino acid residues 198 through 200.

Still further, the mutant RNA polymerase of the second aspect of the present invention can be RNA polymerase derived from K11 phage that has been rendered deficient in the 192 lysine and/or 193 arginine amino acid residue; and rendered deficient in at least one of the three amino acid residues 198 through 200.

The mutant RNA polymerase of the second aspect of the present invention can be, for example, RNA polymerase derived from SP6 phage that has been rendered deficient in the 136 lysine and/or 137 arginine amino acid residues of RNA polymerase.

Further, the mutant RNA polymerase of the second aspect of the present invention can be RNA polymerase derived from SP6 phage that has been rendered deficient in at least one of the four amino acid residues 140 through 143.

Still further, the mutant RNA polymerase of the second aspect of the present invention can be RNA polymerase derived from SP6 phage that has been rendered deficient in the 136 lysine and/or 137 arginine amino acid residue; and rendered deficient in at least one of the four amino acid residues 140 through 143.

The mutant RNA polymerase of the present invention may further possess amino acid substitutions, insertions, and deficiencies other than the above-described substitutions and deficiencies. In such cases, the amino acid substitution, insertion, or deletion is suitably conducted within a scope maintaining the characteristics of the mutant RNA polymerase of the present invention.

The mutant RNA polymerase of the first aspect of the present invention can be constructed from a vector expressing T7 RNA polymerase F644Y/DEL 172-173. This is done on the basis of the plasmid pT7RF644Y that have been employed thus far in transcriptional sequencing, which strongly expresses the T7 RNA polymerase F644Y; and by employing a site-directed mutagenesis method, for example, to prepare DEL 172-173 rendered deficient in amino acid numbers 172 and 173; and constructing the vector.

The mutant RNA polymerase of the second aspect of the present invention can be constructed from a vector expressing T7 RNA polymerase DEL 172-173. This is done on the basis of a plasmid pT7R strongly expressing wild T7 RNA polymerase; and by employing a site-directed mutagenesis method, for example, to prepare DEL 172-173 rendered deficient in amino acid numbers 172 and 173; and constructing the vector.

The method of preparing the mutant RNA polymerase of the present invention will be first described below through the examples of wild T7 RNA polymerase Δ1 (=DEL 172-173) and wild T7 RNA polymerase Δ2 (=DEL 172-173 + DEL 178-180).

A PCR primer not containing the six bases coding for amino acid numbers 172 and 173 is designed, PCR is conducted with plasmid pT7R as template and cycled, the synthesized PCR product is self-ligated to form loops, the loops are introduced into *E. coli* strain JM109, and a transformation is made to obtain a strain resistant to the antibiotic ampicillin. Plasmid is extracted from the transformant. Employing this plasmid as template, a clone that has been rendered deficient in the six bases coding for amino acid numbers 172 and 173 is obtained. During the screening, a clone that is not just deficient in the six pairs coding for amino acid numbers 172 and 173, but that is also deficient in amino acids 178-180, is obtained during the site-directed mutagenesis operation. The former is called T7 RNA polymerase Δ1 and the latter is called T7 RNA polymerase Δ2, and expression plasmids pT7RΔ1 and pT7RΔ2 are constructed to produce them. These plasmids are introduced into *E*. *coli* strain BL21, and IPTG is added to culture solution to activate trc promoter. Mutant polymerase accumulates within the *E*. *coli* bacteria. The method described in Japanese Patent No. 3,172,710 is then used to purify the wild T7 RNA polymerase Δ1 and wild T7 RNA polymerase Δ2 proteins.

In the case of T3 RNA polymerase protein, using the plasmid pT3R expressing this protein as template, pT3RΔ1 deficient in amino acid numbers 173 and 174 corresponding to amino acid numbers 172 and 173 in the T7RNA polymerase above, and pT3RΔ2 corresponding to T7RNA polymerase Δ2, that is, deficient in amino acid numbers 173-174 and 179-181, is constructed. These plasmids can be purified by the same method used to purify the enzyme of T7 RNA polymerase above.

The method of preparing the mutant RNA polymerase of the present invention will be described through the example of wild T7 RNA polymerase Δ3 (DEL 178-180).

A PCR primer not containing the nine bases coding for amino acid numbers 178, 179, and 180 is designed, PCR is conducted with plasmid pT7R as template and cycled, the synthesized PCR product is self-ligated to form loops, the loops are introduced into *E. coli* strain JM109, and a transformation is made to obtain a strain resistant to the antibiotic ampicillin. Plasmid is extracted from the transformant. Employing this plasmid as template, a clone that has been rendered deficient in the nine bases coding for amino acid numbers 178, 179, and 180 is obtained. This clone is called T7 RNA polymerase Δ3, and an expression plasmid pT7RΔ3 producing this clone is built. This plasmid is introduced into *E. coli* strain BL21, and IPTG is added to the culture solution to activate trc promoter. Mutant polymerase accumulates within the *E. coli* bacteria. The method described in Japanese Patent No. 3,172,710 is then used to purify the wild T7 RNA polymerase Δ3 protein.

In the case of T3 RNA polymerase protein, using the plasmid pT3R expressing this protein as template, pT3RΔ3 deficient in amino acid numbers 179 and 181 corresponding to amino acid numbers 178 through 180 in the T7RNA polymerase above, is constructed. This plasmid can be purified by the same method used to purify the enzyme of T7 RNA polymerase above.

The same operation can be used to obtain the mutant RNA polymerase of the present invention for SP6 RNA polymerase and K11 RNA polymerase.

Plasmids expressing T7 RNA polymerase F644Y/Δ1 and T3 RNA polymerase F645Y/Δ1 were deposited on May 30, 2001, in the form of plasmid DNA, pT7 RF644Y/Δ1 as FERM BP-7619 and pT3 RF645Y/Δ1 as FERM BP-7620 at the International Patent Organism Depositary of the National Institute of Advanced Industrial Science and Technology (Chuo No. 6, 1 -1, Higashi 1-chome, Tsukuba-shi, Ibaraki-ken, Japan).

Of the mutant T7 RNA polymerases prepared as set forth above, the enzymatic properties of T7 RNA polymerase F644Y/Δ1 and T7 RNA polymerase F644Y/Δ2 will be described below.

First, Izawa, et al. report that mutant T7 RNA polymerase F644Y increases the ability to incorporate 3'-deoxy nucleotide by about fivefold relative to the corresponding wild form (Izawa, M., et al. J. Biol. Chem., 273 (23): 14242 (1998)). Accordingly, the ability to incorporate 3'-dATP of the wild T7 RNA polymerase, F644Y, and F644Y/Δ1 were compared; there results are given in Table 1. The details of the experiment are given in the Examples.

**Table 1**

| | Relative ability to incorporate 3'-dATP |
|---|---|
| T7 RNA polymerase (wild type) | 1.00 |
| T7 RNA polymerase F644Y | 1.45 |
| T7 RNA polymerase F644Y/Δ1 | 15.5 |

As indicated in Table 1, under the experimental conditions of Table-1, there was an increase of about 1.45-fold in incorporation efficiency between the wild form of T7 RNA polymerase and F644Y, while there was an increase in incorporation efficiency of about 15.5-fold between the wild form and F644Y/Δ1. This was thought to be attributed to increased efficiency of incorporation of 3'-dATP by F644Y, and due to a deficiency in amino acids 172 and 173, 3'-ATP was incorporated, the above-mentioned complex was not formed, separation tended to occur, and the released enzyme was used in the subsequent transcription reaction, enhancing the ability to incorporate. F644Y/Δ2 generated almost the same results. These results indicate that the above-described amino acid deficiency had no effect on the incorporation of 3'-dATP. That is, even when a deficiency in 178-180 was added to T7 RNA polymerase, there was no change in incorporation of 3'-dATP. Thus, there was not thought to be any effect on the incorporation of 3'-dATP. However, when the lysine of 178 and 179 was not present, the PTH signal did not bring about a stop, just as for a 172 and 173 deficiency (Grodberg, J., et al. J. Bacteriol., 170: 1245 (1988)). Thus, it is strongly presumed that just a deficiency in 178 and 179 would function identically to a deficiency in 172 and 173.

The enzymatic properties of T7 RNA polymerase Δ1, T7 RNA polymerase Δ3, T7 RNA polymerase F644Y/Δ1, and T7 RNA polymerase F644Y/Δ3 will be described. The ability to incorporate 3'-dATP of the wild form of T7 RNA polymerase, F644Y, Δ1, Δ3, F644Y/Δ1, and F644Y/Δ3 were compared and the results are given in Table 2. Details of the experiment are given in the Examples.

**Table 2**

| | Relative ability to incorporate 3'-dATP |
|---|---|
| T7 RNA polymerase (wild form) | 1.00 |
| T7 RNA polymerase F644Y | 1.20 ± 0.3 |
| T7 RNA polymerase Δ1 | 1.86 ± 0.3 |
| T7 RNA polymerase Δ3 | 1.90 ± 0.3 |
| T7 RNA polymerase F644Y/Δ1 | 2.30 ± 0.3 |
| T7 RNA polymerase F644Y/Δ3 | 2.00 ± 0.3 |

As shown in Table 2, under the experimental conditions of Table 2, while there was an increase in incorporation efficiency of 1.20 ± 0.3 fold between the wild form of T7 RNA polymerase and F644Y, there were increases in incorporation efficiency of 1.86 ± 0.3 fold and 1.90 ± 0.3 fold between the wild form and Δ1 and Δ3, respectively. Further, there were increases in incorporation efficiency of 2.30 ± 0.3 fold and 2.00 ± 0.3 fold between the wild form and F644Y/Δ1 and F644Y/Δ3, respectively. This was thought to be attributed to deficiency in amino acids 172 and 173, and deficiency in amino acids 172 and 173 as well as 178 through 180 causing 3'-ATP to be incorporated without forming the above-described complex, separation tending to occur, and the released enzyme being used in the next transcription reaction, thus achieving the effect of enhancing incorporation ability. Further, it was presumed that the addition of F644Y further increased the efficiency with which 3'-dATP was incorporated.

### DNA Sequencing Methods

The mutant RNA polymerase of the present invention can be used in methods of DNA sequencing employing an RNA polymerase transcription reaction. This sequencing method comprises the steps of (1) obtaining nucleic acid transcription reaction product employing mutant RNA polymerase, template DNA having a promoter sequence for this RNA polymerase, and substrates of the above-described RNA polymerase, (2) separating the nucleic acid transcription reaction product obtained, and (3) reading the sequence of the nucleic acid from the separated fractions obtained.

The method of enzymatically synthesizing nucleic acid transcription reaction product using RNA polymerase with template in the form of a DNA fragment containing a promoter sequence for RNA polymerase, the method of separating nucleic acid transcription product, and the method of reading the sequence of nucleic acid from the separated fractions are in principle all known. Accordingly, it is basically possible to suitably employ any known method as well as known conditions, devices, and the like in this regard.

### (1) The step of obtaining nucleic acid transcription reaction product

With the exception that a promoter sequence for RNA polymerase is contained in the DNA fragment used as template, there are no specific limits. For example, DNA fragments containing promoter sequence can be the DNA products of amplification by polymerase chain reaction. Further, it is possible to conduct the nucleic acid transcription reaction in the method of the present invention without removing the primer employed in the polymerase chain reaction and/or 2' deoxyribonucleoside 5' triphosphate and/or derivatives thereof from the amplified DNA product. The widely employed PCR method can be used without modification as the above polymerase chain reaction to amplify DNA. The DNA fragments containing a promoter sequence may be DNA fragments that are cloned using a suitable host after ligating the promoter sequence to the DNA fragment to be amplified. That is, neither the DNA sequence to be amplified, the primer, nor the amplification conditions are specifically limited.

For example, the reaction system of the polymerase chain reaction used to amplify the DNA fragment containing the promoter sequence may be in the form of a 20 µL volume comprising 10 to 50 ng of genomic DNA or 1 pg of cloned DNA, 10 µ moles of each primer, and 200 µmoles of each 2' deoxyribonucleoside 5' triphosphate (dATP, dGTP, dCTP, dTTP), employing a DNA polymerase such as Taq polymerase.

However, it is necessary for at least one of the primers for the polymerase chain reaction or the amplified insert DNA to contain the promoter sequence of RNA polymerase, described further below. In direct transcriptional sequencing, two primers, one of which has a phage promoter sequence, are employed in PCR, or the phage promoter sequence is imparted to the insert DNA that is being amplified, so that the PCR product obtained can be used in *in vitro* transcription employing RNA polymerase activated by that promoter.

The promoter sequence for the RNA polymerase can be suitably selected based on the type of RNA polymerase being employed. By introducing two promoters derived from phages - for example, two promoters specifically recognized by T7 and T3 phages, respectively - into the strands of the template, simple selection of any RNA polymerase at the time of the transcriptional sequencing reaction makes it possible to analyze the sequence data at the two ends of the target DNA.

In this method, the nucleic acid transcription product of RNA transcription product is synthesized from a DNA fragment containing the promoter sequence. Since the DNA fragment contains a promoter sequence for RNA polymerase, this promoter sequence activates the mutant RNA polymerase, synthesizing the nucleic acid transcription product of the RNA transcription product.

The synthesis of the nucleic acid transcription product of the RNA transcription product employs a substrate comprising at least 3'-deoxynucleotide or fluorescent-labeled 3'-deoxynucleotide (3'- dNTP derivative). More specifically, the substrate is employed in the form of a ribonucleoside 5' triphosphate (NTP) comprised of ATP, GTP, CTP, and UTP or derivatives thereof, and one or more 3' dNTP derivative. In the present Specification, the 3' dNTP derivative is employed collectively for 3'-dATP, 3'-dGTP, 3'-dCTP, 3'-dUTP, and their derivatives. Including the case where a portion is a derivative such as ATP, at least four ribonucleotide 5' triphosphate (NTP) compounds with different bases are required for the synthesis of a transcription product. However, it is also possible to employ two or more compounds comprising the same base.

Incorporating a 3' dNTP derivative on the 3' terminal of the RNA or nucleic acid that is the product of transcription causes the 3' hydroxy group to drop off, blocking RNA or nucleic acid synthesis. As a result, RNA or nucleic acid fragments of varying length having 3' dNTP derivatives on the 3' terminal are obtained. The four types of 3' dNTP derivatives of differing bases thus yield corresponding ribonucleoside analogs. By preparing these four types of ribonucleoside analogs, RNA or nucleic acid sequencing is possible (Vladimir D. Axelred et al. (1985) Biochemistry Vol. 24, 5716-5723).

One or more 3' dNTP derivatives may be employed in a single nucleic acid transcription reaction. When just one 3' dNTP derivative is employed to conduct a single nucleic acid transcription reaction, the nucleic acid transcription reaction can be conducted four times to obtain four transcription products with different 3' dNTP derivate bases on the 3' terminal. In a single nucleic transcription reaction, it is possible to obtain a transcription product in the form of a mixture of various RNA or nucleic acid fragments of differing molecular weight with an identical 3' dNTP derivative on the 3' terminal. It is possible to independently separate (described further below) and read the sequences of the four transcription products obtained. It is also possible to mix two or more of four transcription products, separate the mixture, and read the sequence.

When employing two or more 3' dNTP derivatives simultaneously in a single nucleic acid transcription reaction, two or more transcription products with 3' dNTP derivative bases on the 3' terminal are contained in a single reaction product. This can be separated (described further below) and the sequence read. The simultaneous use of two or more 3' dNTP derivatives in a nucleic acid transcription reaction is desirable because it permits a reduction in the number of nucleic acid transcription reaction operations.

In particular, it is desirable for nucleic acid transcription of RNA or the like to be terminated by four types of 3' dNTP derivatives comprising different bases, separation to be conducted, and the sequence of the four bases to be read at once (simultaneously).

### (2) The step of separating the nucleic acid transcription reaction product

Next, the RNA or nucleic acid transcription product is separated. This separation can be suitably conducted by a method of separating by molecular weight the multiple product molecules of differing molecular weight contained in the transcription product. An example of such a method is electrophoresis. In addition, HPLC and the like may be employed.

The conditions employed in electrophoresis or the like are not specifically limited and the usual method may be employed. The RNA or nucleic acid sequence can be read from the bands (RNA or nucleic acid ladder) obtained by subjecting the transcription product to electrophoresis.

### (3) The step of reading the nucleic acid sequence

The RNA or nucleic acid ladder can be read by labeling the 3'-dNTP derivatives. The RNA or nucleic acid ladder can also be read by labeling the ribonucleoside 5' triphosphates (NTPs). Examples of labels are radioactive and stable isotopes and fluorescent labels.

Specifically, labeled 3'-dNTP derivatives, more specifically, labeled 3'-dATP, 3'-dGTP, 3'-dCTP, and 3'-dUTP are employed and the radioactivity or detection of stable isotopes or fluorescence of the bands obtained by subjecting the transcription product to electrophoresis can be used to read the sequence of the transcription products. Labeling the 3' dNTP derivatives in this manner facilitates measurement of the radioactive intensity or fluorescent intensity between bands without dispersion. The ladder generating radioactivity, stable isotopes, or fluorescence can be suitably detected with a DNA sequencing device.

It is also possible to read the sequence of the transcription product by employing ATP, GTP, CTP, and UTP labeled with radioactivity, stable isotopes, or fluorescence; conducting electrophoresis to obtain bands; and determining the radioactivity, stable isotopes, or fluorescence of the bands.

It is further possible to employ 3'-dATP, 3'-dGTP, 3'-dCTP, and 3'-dUTP labeled with a different type of fluorescence, subject a mixture of various transcription product fragments having a different type of label and terminal in the form of 3'-dATP, 3'-dGTP, 3'-dCTP or 3'-dUTP to electrophoresis; and read the sequence of the RNA or nucleic acid by detecting the four types of fluorescence of the bands obtained.

In this method, four types of 3'-dNTP are each labeled with a different type of fluorescence. When this is done, subjecting a mixture of the four types of transcription product having different 3' terminals to electrophoresis to obtain bands generating fluorescence based on the 3'-dNTPs with four different 3' terminals and identifying the differences in fluorescence permits the reading of the sequence of four types of RNA or nucleic acid at once.

The use of the 3'-deoxyribonucleotide derivative described in Japanese Unexamined Patent Publication (KOKAI) Heisei No. 11-80189 and WO99/02544, for example, as the 3'-dNTP is desirable.

The effect in base sequence analysis of actual DNA in transcriptional sequencing employing the mutant RNA polymerase of the present invention will be described next.

The reaction conditions conformed to the method described by Sasaki et al. (Sasaki, N., et al., Gene, 222 (1): 17-24 (1998)). That is, employing plasmid pBluescriptII DNA having a promoter for T7RNA polymerase as template, an *in vitro* transcription reaction was conducted for 60 min while maintaining 37°C. The unreacted dye terminator remaining in the reaction product following the reaction was removed, and analysis was conducted with an ABI PRISM 377XL DNA sequencer. Fig. 4 shows the typical results of this experiment compared to a gel image. As a result, electrophoresis of T7 RNA polymerase F644Y resulted in a high level of noise in the separation of RNA fragments terminated with specific bases. The individual electrophoretic lanes were found to present continuous rod-shaped images. However, when T7 RNA polymerase F644Y/Δ2 was employed, individual sharp RNA separation images were obtained for the transcription products and there was little noise. This indicated that accurate base calls were possible, and as a result, accurate base sequence analysis was possible.

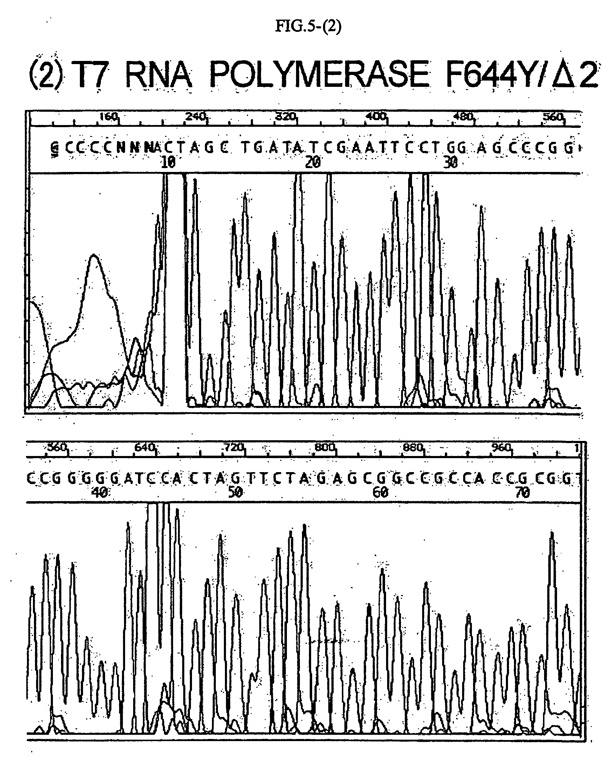

Base calls were prepared from these results. Portions of a base sequence that was read are presented in Fig. 5 and 6. T7 RNA polymerase F644Y/Δ2 was found to permit more accurate base sequence analysis than T7 RNA polymerase F644Y. Similar results were obtained for F644Y/Δ3 and T7 RNA polymerase Δ1, Δ2, and Δ3 without the F644Y mutation.

### Examples

The present invention is described in greater detail below through examples.

### [Example 1]

### The introduction of amino acid deficiencies into T7 RNA polymerase F644Y and T3 RNA polymerase F645Y enzyme and purification of mutant RNA polymerases thereof

A plasmid expressing mutant enzyme rendered deficient in amino acid numbers 172 and 173 of T7 RNA polymerase F644Y was prepared using a plasmid pT7RF644Y (Japanese Unexamined Patent Publication (KOKAI) Heisei No. 11-75867, Bioresearch International Depository No. FERM-BP-5998) producing T7 RNA polymerase F644Y to conduct site-directed mutagenesis method based on PCR.

A plasmid expressing mutant enzyme rendered deficient in amino acid numbers 173 and 174 of T3 RNA polymerase F645Y was prepared using a plasmid pT3RF645Y producing T3 RNA polymerase F645Y to conduct site-directed mutagenesis method based on PCR.

The T3 RNA polymerase F645Y was prepared in the following manner.

T3 phage having *E. coli* as host was inoculated into *E. coli* C600, T3 phage was prepared by the usual method from the bacteriolytic solution, and finally, a protein removal treatment was conducted, yielding genome DNA derived from T3 phage. Since the T3 RNA polymerase gene is contained in this genome DNA, a plasma was prepared for the amplification of the T3 RNA polymerase gene by consulting the data recorded in McGraw, N.J., et al. Nucleic Acids Res. 13 (18): 6753 (1985), and PCR amplification was conducted. The DNA fragments were digested with the restriction enzyme NcoI, 1 % agarose electrophoresis was conducted, the targeted DNA fragments were cut out of the agarose, and purification was conducted with a Gene Pure Kit (Nippon Gene). This was digested with NcoI and linked to dephosphorized expression vector pTrc99A (Pharmacia Biotech) to construct T3 RNA P/pTrc99A strongly expressing T3 RNA polymerase. This plasmid was confirmed to express large quantities of T3 RNA polymerase by the usual method by adding IPTG to the culture medium.

Next, expression plasmid producing mutant T3 RNA polymerase F645Y was constructed with plasmid T3 RNA P/pTrc99A as set forth below. T3 RNA P/pTrc99A was employed as template. Using PCR primer for mutant-introduction, PCR was conducted with Pfu DNA polymerase (Stratagene). These PCR fragments were purified by 1 % agarose electrophoresis, after which a ligation reaction was conducted with T4 DNA ligase at 16°C maintained for 16 hours. The reaction product was introduced into *E. coli* JM109, screening was conducted for clones incorporating the mutation, and the base sequence was determined to confirm that the mutation had been incorporated. A plasmid T3 RNA P(F645Y)/pTrc99A producing mutant T3 RNA polymerase F645Y was constructed. The mutant T3 RNA polymerase F645Y was purified by the method described in Japanese Unexamined Patent Publication (KOKAI) Heisei No. 11-075867 using the plasmid T3 RNA P/pTrc99A.

The method of preparing amino acid-deficient mutant enzymes is described in detail below.

Plasmid pT7RF644Y DNA expressing T7 RNA polymerase F644Y was employed as template and PCR was conducted with primer T7RNAP-ΔN (5'-TACAAGAAAGCATTTATGCAAGTTGTCGAGG-3') (Sequence Number 1) and primer T7RNAP-ΔC (5'-GACGTGCCCTACGTTGAGTTGTTCCTCAAC-3') (Sequence Number 2). For T3 RNA polymerase, pT3RF645Y DNA was employed as template with primer T3RNAP-ΔN (5'-TACAGAAAGCATTTATGCAGGTG-GTCGAGG-3') (Sequence Number 3) and primer T3RNAP-ΔC (5'-GACTTG-CCCGTGGTTAAGCTGTTCCTCAAC-3') (Sequence Number 4). Both were design primers; primer T7 RNAP-ΔC deficient in amino acids 172 and 173 in the case of T7, and primer T3 RNAP-ΔC deficient in amino acids 173 and 174 in the case of T3 (Fig. 3). When PCR was conducted for the above combinations, since looped plasmids were employed as template DNA in both cases, the PCR product obtained was nearly identical in length to the template plasmid DNA employed. The PCR fragments were subjected to 1 % agarose electrophoresis and purified. The fragments were then relooped (by self-ligation) with T4 DNA ligase, and introduced into *E. coli* JM109. Multiple colonies were obtained by cultivation on agar sheets containing the antibiotic ampicillin. Several of these colonies were selected and cultured. The plasmid DNA was extracted and base sequencing of regions coding for multiple RNA polymerases of plasmid DNA was conducted to determine whether or not the mutations had been correctly introduced and, in this introduction, whether or not any change had occurred in the regions coding for RNA polymerases. For T7 RNA polymerase F644Y, as expected, this operation yielded multiple plasmids deficient in amino acids 172 and 173 and plasmids deficient in amino acids 178 through 180 in addition to being deficient in amino acids 172 and 173. The former were called pT7RF644Y/Δ1 (Fig. 3(1)) and the latter pT7RF644Y/Δ2.

In a manner identical to the results of introducing deficiencies into T7 RNA polymerase above, a mutant plasmid deficient in amino acids 173 and 174 and a mutant plasmid deficient in amino acids 179 through 181 were obtained as expected from T3 RNA polymerase F645Y. The former was called pT3RF645Y/Δ1 (Fig. 3(2)) and the latter pT3RF645Y/Δ2.

It was possible to induce expression of each of the four plasmids that were prepared here by culturing *E. coli* BL21 containing the respective plasmid and adding isopropyl-beta-thiogalactopyranosyl (IPTG).

All of the enzymes were basically purified by the same method from *E*. *coli* BL21 containing these plasmids. Although the chromatographic operation varied some based on the mutation site and quantity expressed, the methods described in Japanese Unexamined Patent Publication (KOKAI) Heisei No. 11-75867 were employed for purification and testing of purity and activity. The various RNA polymerases obtained by these methods exhibited an approximately one-third reduction in expression level relative to the original plasmids in which deficiency mutations had not been introduced. However, purification yielding enzymes of a quality fully adequate for transcriptional sequencing was possible.

### The introduction of amino acid deficiencies into wild T7 RNA polymerase and wild T3 RNA polymerase and the purification of mutant RNA polymerases thereof

The method of preparing mutant enzymes rendered deficient in amino acids is described in detail below.

Plasmid pT7R DNA expressing T7 RNA polymerase was employed as template and PCR was conducted with primer T7RNAP-ΔN (5'-TACAAGAAAGCATTTATGC-AAGTTGTCGAGG-3') (Sequence Number 1) and primer T7RNAP-ΔC (5'-GACGTGCCCTACGTTGAGTTGTTCCTCAAC-3') (Sequence Number 2). For T3 RNA polymerase, pT3R DNA was employed as template with primer T3RNAP-ΔN (5'-TACAAGAAAGCATTTATGCAGGTGGTCGAGG-3') (Sequence Number 3) and primer T3RNAP-ΔC (5'-GACTTGCCCGTGGTTAAGCTGTTCCTCAAC-3') (Sequence Number 4). Both were design primers; primer T7 RNAP-ΔC deficient in amino acids 172 and 173 in the case of T7 and primer T3 RNAP-ΔC deficient in amino acids 173 and 174 in the case of T3 (Fig. 3). When PCR was conducted for the above combinations, since looped plasmids were employed as template DNA in both cases, the PCR product obtained was nearly identical in length to the template plasmid DNA employed. The PCR fragments were subjected to 1 % agarose electrophoresis and purified. The fragments were then relooped (by self-ligation) with T4 DNA ligase, and introduced into *E. coli* JM109. Multiple colonies were obtained by cultivation on agar sheets containing the antibiotic ampicillin. Several of these colonies were selected and cultured. The plasmid DNA was extracted and base sequencing of regions coding for multiple RNA polymerases of plasmid DNA was conducted to determine whether or not the mutations had been correctly introduced and, in this introduction, whether or not any change had occurred in the regions coding for RNA polymerases. For T7 RNA polymerase, as expected, this operation yielded multiple plasmids deficient in amino acids 172 and 173 and plasmids deficient in amino acids 178 through 180 in addition to being deficient in amino acids 172 and 173. The former were called pT7R/Δ1 and the latter pT7RΔ2.

In a manner identical to the results of introducing deficiencies into T7 RNA polymerase above, a mutant plasmid deficient in amino acids 173 and 174 and a mutant plasmid deficient in amino acids 179 through 181 were obtained as expected from T3 RNA polymerase. The former was called pT3RΔ1 and the latter pT3RΔ2.

When PCR was conducted in the same manner as set forth above employing primer T7 RNAP-Δ3C, a primer designed to impart deficiency in amino acids 178 through 180 in T7, and employing primer T3 RNAP-Δ3C, a primer designed to impart deficiency in amino acids 179 through 181 in T3, a PCR product substantially identical in length to the template plasmid DNA employed was obtained. These PCR products were purified and the lack of change in regions coding for the RNA polymerase was confirmed. As expected, this operation yielded plasmid pT7RΔ3 deficient in amino acids 178 through 180 for T7 RNA polymerase, and plasmid pT3RΔ3 deficient in amino acids 179 through 181 for T3 RNA polymerase.

It was possible to induce expression of each of the six plasmids that were prepared by culturing *E. coli* BL21 containing the respective plasmid and adding isopropyl-beta-thiogalactopyranosyl (IPTG).

All of the enzymes were basically purified by the same method from *E. coli* BL21 containing these plasmids. Although the chromatographic operation varied some based on the mutation site and quantity expressed, the methods described in Japanese Unexamined Patent Publication (KOKAI) Heisei No. 11-75867 were employed for purification and testing of purity and activity. The various RNA polymerases obtained by these methods exhibited a substantially one-third reduction in expression level relative to the original plasmids in which deficiency mutations had not been introduced. However, purification yielding enzymes of a quality fully adequate for transcriptional sequencing was possible.

### [Example 2]

### The properties of deficient mutant RNA polymerase

The ability to incorporate 3'-dATP of the Δ type enzyme of purified T7 RNA polymerase was compared to that of the wild type and F644Y mutation.

### Test (1)

In this method, 3'-dATP was added during an *in vitro* transcription reaction and the synthesis level was determined by comparison with synthesis impairment. This will be described in greater detail. The template employed was pBluescriptII (Stratagene) pretreated with the restriction enzyme ScaI (made by Nippon Gene). The reaction conditions were: 40 mM Tris-HCl, pH 8.0, 25 mM NaCl, 8 mM MgCl₂, 2 mM spermidine-(HCl)₃, and 5 mM DTT. The concentration of nucleotides ATP, CTP, GTP, and UTP was 0.5 mM. Of these, ATP was replaced with 3'-dATP (Sigma) in ranges of 0.006 mM, 0.008 mM, 0.012 mM, and 0.024 mM. The above purified enzymes were added to 20 µL of this solution and the reaction was conducted for 30 to 60 min at 37°C. At the conclusion of the reaction, 4 µL of 5 x gel loading buffer was added (15 % Ficoll, 0.1 % BPB, 0.1 % XC, 0.5 % SDS, and 0.1 M EDTA). The entire solution was then subjected to electrophoresis in a 1 % modified agarose gel containing formaldehyde. At the conclusion of electrophoresis, 10,000-fold dilution was conducted with SYBER Green II (made by Molecular Probe Co.) and dying was conducted for 20 to 40 min according to a protocol of addition to the product. A Fluoro S Multiimager (BioRad Co.) was then employed to irradiate ultraviolet radiation and pick up the fluorescent image. Multianalyst, an attached image analyzing software package, was used to determine the amount of RNA that had been synthesized from the image picked up. The various values of the results were compared. An increase in ability to incorporate 3'-dATP should result in a drop in the synthesis level. Accordingly, a comparison was made with a control, and the inverse thereof was employed as indicator of how readily 3'-dATP was incorporated. The results indicated that when the 3'-dATP concentration was 0.024 mM, taking the wild form of T7 RNA polymerase as 1, the relative ability to incorporate 3'-dATP was 1.45 for F644Y and 15.5 for F644Y/Δ1 (Table 1 above). This result suggests that T7 RNA polymerase F644Y/Δ1 was an enzyme having optimal properties for transcriptional sequencing.

### Test (2)

In this method, 3'-dATP was added during an *in vitro* transcription reaction and the synthesis level was determined by comparison with synthesis impairment. This will be described in greater detail. The template employed was pBluescriptII (Stratagene) pretreated with the restriction enzyme ScaI (made by Nippon Gene). The reaction conditions were: 40 mM Tris-HCl, pH 8.0, 25 mM NaCl, 8 mM MgCl₂, 2 mM spermidine-(HCl)₃, and 5 mM DTT. The concentration of ribonucleotides rATP, rCTP, rGTP, and rUTP was 0.5 mM. A 0.1 mM quantity of Biotin-labeled CTP was added and 3'-dATP was added in ranges of 0.00125 mM, 0.0025 mM, and 0.005 mM. A 25 U quantity of enzymes purified as set forth above were added to 10 µL of this solution and the reaction was conducted for 60 min at 37°C. At the conclusion of the reaction, 1 µL of 10 x gel loading buffer (1 mM EDTA, 1 % SDS, 50 % glycerol, and 0.05 % BPB) was added. The entire solution was then subjected to electrophoresis in a 1 % modified formamide gel. At the conclusion of electrophoresis, northern hybridization was conducted, the synthesized product was transcribed onto a nylon membrane, and signal detection was conducted based on a chemoluminescent substrate according to a protocol of anti-SA-AP antibody (made by Oriental Yeast Co., diluted 20,000-fold) and CDP-Star™ (made by Roche Co.). A Fluoro S Multiimager (BioRad Co.) and Multianalyst, an attached image analyzing software package, were then employed to determine the level of the signal detected.
As shown in Table 2, the results indicated that when the 3'-dATP concentration was 0.00125 mM, taking the ability of the wild form to incorporate 3'-dATP as 1, the values of the various mutant RNA polymerases were 1.20 for F644Y, 1.86 for Δ1, 1.90 for Δ3, 2.30 for F644Y/Δ1, and 2.00 for F644Y/Δ3 (average of three runs).

### [Example 3]

### A comparison of the ability to incorporate fluorescent-labeled 3'-deoxynucleotide of deficient mutant RNA polymerase

A comparison was conducted of the ability to incorporate florescent-labeled 3'-deoxynucleotide of Δ-type enzyme of purified T7 RNA polymerase.

This method was conducted in accordance with the method of Sasaki et al. (Sasaki, N., et al. Gene, 222(1): 17-24 (1998)). In greater detail, pBluescriptII DNA was employed as template. A solution obtained by mixing Bodipy(R6G)-3'dATP, Bodipy(FL)-3'dGTP, Bodipy(581/591)-3'dCTP, Bodipy(564/570)-3'dUTP, ATP, GTP, CTP, and UTP in prescribed proportions was employed to conduct reactions differing only with regard to the T7 RNA polymerase employed. The enzymes employed were 100 units of T7 RNA polymerase (wild type) and T7 RNA polymerase F644Y; and 50 units of T7 RNA polymerase F644Y/Δ1. The reaction product was subjected to electrophoresis with an ABI 377 XL Sequencer. The average value of the fluorescent signal intensity was compared for each nucleotide. The results are given in Table 3.

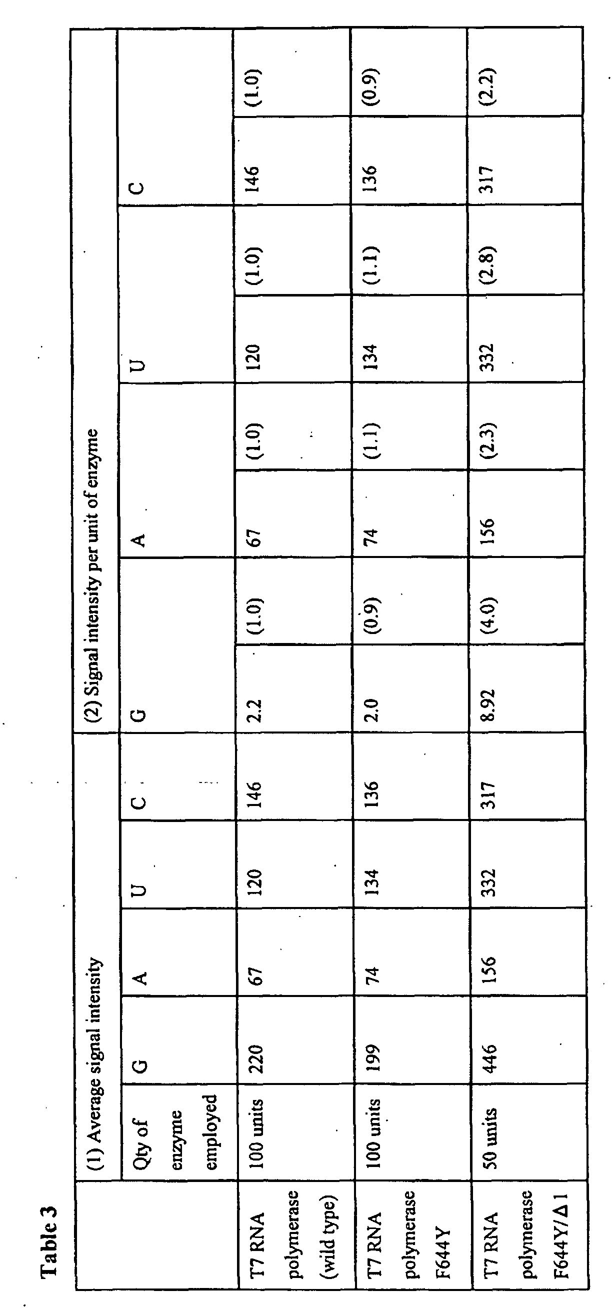

In the table, column (1) indicates the average intensity of the fluorescent signal displayed on the ABI 377 XL Sequencer in the present experiment. Column (2) is a conversion of the value of (1) per unit of enzyme. Numbers shown in parentheses denote values relative to a value of "1" for the signal intensity of wild enzyme. These results reveal that although the fluorescent intensity of T7 RNA polymerase F644Y/A 1 was varied based on the nucleotide, it was found to be 2 to 4 times more efficient than that of the wild type and F644Y in incorporating fluorescent-labeled 3'-deoxynucleotide. This result matches the result and trend of the above incorporation test of 3'-dATP without fluorescent label. These results were virtually identical for the wild type and F644Y. Since the ABI 377 XL Sequencer is not a device for quantitative analysis, and operations is conducted on an extremely small amount of samples, large errors are to be expected. However, the effect of Δ1 was adequately revealed in this detection system.

### [Reference Examples]

### Example of transcriptional sequencing employing mutant enzyme

T7 RNA polymerase F644Y/Δ2 and T7 RNA polymerase F644Y that had been prepared and purified by the above-described methods were employed in transcriptional sequencing and the results were compared.

The transcriptional sequencing reaction was conducted in accordance with the method of Sasaki et al. (Sasaki, N., et al. Gene, 222(1): 17-24 (1998)). In greater detail, pBluescriptII DNA was employed as template. Solutions obtained by mixing 3'-dNTP derivatives in the form of Bodipy(R6G)-3'dATP, Bodipy(FL)-3'dGTP, Bodipy(581/591)-3'dCTP, Bodipy(564/570)-3'dUTP, and ATP, GTP, CTP, and UTP were suitably diluted and admixed. The solutions were reacted with 50 units of T7 RNA polymerase F644Y and analysis was conducted with an ABI 377 DNA XL Sequencer. An optimal solution permitting a base call of about 500 bases was thus prepared. An *in vitro* transcription reaction was conducted for 60 min while maintaining a temperature of 37° with this nucleotide optimal solution and 50 units of T7 RNA polymerase F644Y/Δ2. Following the reaction, unreacted dye terminator remaining in the reaction product was eliminated and gel filtration was conducted with a Sephadex G-50 column (made by Amersham Pharmacia Biotech) to purify the transcription product. The transcription product was then solidified by evaporation using a centrifugal concentrator. The dried product was dissolved in 6 µL of formamide/EDTA/Bluedextran loading buffer according to Version 1.0 of the instruction manual of an ABI Prism 377XL DNA Sequencer made by Perkins Elmer Japan, Ltd. and 2 µL thereof were analyzed by the ABI Prism 377XL DNA Sequencer. The typical results of this experiment are given in Fig. 4 as a comparison of gel images. As a result, although electrophoresis of T7 RNA polymerase F644Y separated RNA fragments terminated by specific bases, the noise level was high and each electrophoretic lane was found to present a continuous, rod-shaped image. However, when T7 RNA polymerase F644Y/Δ2 was employed, individual separation images of the RNA of the transcription product were clear and noise was low. This indicated that accurate base calls were possible, and as a result, it was readily presumed that accurate base sequence analysis would also be possible. Since noise was low in base sequence analysis, this property made it possible to anticipate that the length of bases that it would be possible to read in a single reaction with an accurate base call would increase.

Figs. 5 and 6 are comparisons of base calls of a portion of the electrophoretic images of the above gel images. These results indicate that by employing the current Δ-type enzyme in transcription sequencing, noise was low. Even when 3'-dNTP was incorporated, it was efficiently recycled in transcription. Thus, this enzyme was optimal for transcriptional sequencing.

### Industrial Applicability

The mutant RNA polymerase of the present invention permits a reduction in noise in the reaction product when employed in transcriptional sequencing. When employing a capillary sequencer with the object of conducting a large amount of sequencing, this characteristic permits, for example, template preparation by directly adding a PCR product to the reaction solution, thus saving time and money. It also affords advantages in the form of greater precision of analysis data and the reading of longer strands. The present invention provides a method that is particularly valuable in the fields of genetic analysis and genetic diagnosis involving base sequencing. Since no purification of the PCR template is necessary and it can be used directly, an extremely efficient base sequencing method for use in large amounts of sequencing is achieved.

## Claims

1. Mutant RNA polymerase in which at least one of the amino acids of wild RNA polymerase has been substituted and in which at least one amino acid has been rendered deficient, **characterized in that** the substitution and deficiency of said amino acids is conducted in such a manner as to enhance the ability to incorporate 3'-deoxy nucleotide as substrate relative to that of the corresponding wild RNA polymerase.

2. Mutant RNA polymerase for use in transcriptional sequencing in which at least one of the amino acids of wild RNA polymerase has been rendered deficient, **characterized in that** the deficiency of said amino acid is conducted in such a manner as to enhance the ability of incorporating 3'-deoxy nucleotide as substrate relative to that of the corresponding wild RNA polymerase.

3. The mutant RNA polymerase of claim 1 or 2, wherein the 3'-deoxy nucleotide is either 3'-deoxynucleotide having or not having a label.

4. The mutant RNA polymerase of claim 1 or 2, wherein the 3'-deoxynucleotide having a label is 3'-deoxynucleotide having a fluorescent label.

5. The mutant RNA polymerase of any of claims 1 to 4, wherein the deficiency of an amino acid is effected on a basic amino acid present in a region upon which protease acts.

6. The mutant RNA polymerase of any of claims 1 to 4, wherein the deficiency of an amino acid is effected on a basic amino acid present in a region upon which protease of *E*. *coli* acts.

7. The mutant RNA polymerase of claim 5 or 6, wherein the basic amino acid is lysine and/or arginine.

8. The mutant RNA polymerase of any of claims 1 to 7, wherein the wild RNA polymerase is RNA polymerase derived from T7 phage, T3 phage, SP6 phage, or K11 phage.

9. The mutant RNA polymerase of any of claims 1 to 4, wherein the wild RNA polymerase is RNA polymerase derived from T7 phage, the deficiency of an amino acid is effected in the region containing at least one amino acid selected from the group of amino acid 172, 173, 178, 179 and 180.

10. The mutant RNA polymerase of any of claims 1 to 4, wherein the wild RNA polymerase is RNA polymerase derived from T3 phage, the deficiency of an amino acid is effected in the region containing at least one amino acid selected from the group of amino acid 173, 174, 179, 180 and 181.

11. The mutant RNA polymerase of any of claims 1 to 4, wherein the wild RNA polymerase is RNA polymerase derived from K11 phage, the deficiency of an amino acid is effected in the region containing at least one amino acid selected from the group of amino acid 192, 193, 198, 199, and 200.

12. The mutant RNA polymerase of any of claims 1 to 4, wherein the wild RNA polymerase is RNA polymerase derived from SP6 phage, the deficiency of an amino acid is effected in the region containing at least one amino acid selected from the group of amino acid 136, 137, 140, 141, 142, and 143.

13. The mutant RNA polymerase of any of claims 1 to 12, wherein the number of amino acids rendered deficient is from 1 to 10.

14. The mutant RNA polymerase of any of claims 1 to 12, wherein the number of amino acids rendered deficient is from 1 to 7.

15. The mutant RNA polymerase of any of claims 1 to 12, wherein the number of amino acids rendered deficient is from 1 to 5.

16. The mutant RNA polymerase of any of claims 1 to 12, wherein the number of amino acids rendered deficient is 1, 2, or 3.

17. The mutant RNA polymerase of any of claims 1 to 12, wherein the mutant RNA polymerase further possesses amino acid substitutions, insertions, and deficiencies other than the above-described substitutions and/or deficiencies.

18. The mutant RNA polymerase of claim 1, wherein the mutant RNA polymerase is RNA polymerase obtained by substituting tyrosine for the phenylalanine at amino acid residue 644 or 667, or both 664 and 667, of RNA polymerase derived from T7 phage, and deleting the 172 lysine and/or 173 arginine amino acid residue.

19. The mutant RNA polymerase of claim 1, wherein the mutant RNA polymerase is RNA polymerase obtained by substituting tyrosine for the phenylalanine at amino acid residue 644 or 667, or both 664 and 667, of RNA polymerase derived from T7 phage, and deleting at least one of the amino acid residues from among the three amino acid residues 178 through 180.

20. The mutant RNA polymerase of claim 1, wherein the mutant RNA polymerase is RNA polymerase obtained by substituting tyrosine for the phenylalanine at amino acid residue 644 or 667, or both 664 and 667, of RNA polymerase derived from T7 phage; deleting the 172 lysine and/or 173 arginine amino acid residue; and deleting at least one of the amino acid residues from among the three amino acid residues 178 through 180.

21. The mutant RNA polymerase of claim 1, wherein the mutant RNA polymerase is RNA polymerase obtained by substituting tyrosine for the phenylalanine at amino acid residue 645 or 668, or both 645 and 668, of RNA polymerase derived from T3 phage, and deleting the 173 lysine and/or 174 arginine amino acid residue.

22. The mutant RNA polymerase of claim 1, wherein the mutant RNA polymerase is RNA polymerase obtained by substituting tyrosine for the phenylalanine at amino acid residue 645 or 668, or 645 and 668, of RNA polymerase derived from T3 phage, and deleting at least one of the amino acid residues from among the three amino acid residues 179 through 181.

23. The mutant RNA polymerase of claim 1, wherein the mutant RNA polymerase is RNA polymerase obtained by substituting tyrosine for the phenylalanine at amino acid residue 645 or 668, or 645 and 668, of RNA polymerase derived from T3 phage; deleting the 173 lysine and/or 174 arginine amino acid residue; and deleting at least one of the amino acid residues from among the three amino acid residues 179 through 181.

24. The mutant RNA polymerase of claim 1, wherein the mutant RNA polymerase is RNA polymerase obtained by substituting tyrosine for the phenylalanine at amino acid residue 690 of RNA polymerase derived from K11 phage and deleting the 192 lysine and/or 193 arginine amino acid residue.

25. The mutant RNA polymerase of claim 1, wherein the mutant RNA polymerase is RNA polymerase obtained by substituting tyrosine for the phenylalanine at amino acid residue 690 of RNA polymerase derived from K11 phage, and deleting at least one of the amino acid residues from among the three amino acid residues 198 through 200.

26. The mutant RNA polymerase of claim 1, wherein the mutant RNA polymerase is RNA polymerase obtained by substituting tyrosine for the phenylalanine at amino acid residue 690 of RNA polymerase derived from K11 phage; deleting the 192 lysine and/or 193 arginine amino acid residue; and deleting at least one of the amino acid residues from among the three amino acid residues 198 through 200.

27. The mutant RNA polymerase of claim 1, wherein the mutant RNA polymerase is RNA polymerase obtained by substituting tyrosine for the phenylalanine at amino acid residue 670 of RNA polymerase derived from SP6 phage, and deleting the lysine 136 and/or arginine 137 amino acid residue.

28. The mutant RNA polymerase of claim 1, wherein the mutant RNA polymerase is RNA polymerase obtained by substituting tyrosine for the phenylalanine at amino acid residue 670 of RNA polymerase derived from SP6 phage, and deleting at least one of the basic amino acids among the four amino acid residues 140 through 143.

29. The mutant RNA polymerase of claim 1, wherein the mutant RNA polymerase is RNA polymerase obtained by substituting tyrosine for the phenylalanine at amino acid residue 670 of RNA polymerase derived from SP6 phage; deleting the lysine 136 and/or arginine 137 amino acid residue; and deleting at least one of the basic amino acids among the four amino acid residues 140 through 143.

30. The mutant RNA polymerase of claim 2, wherein the mutant RNA polymerase is RNA polymerase rendered deficient in the 172 lysine and/or 173 arginine amino acid residues of RNA polymerase derived from T7 phage.

31. The mutant RNA polymerase of claim 2, wherein the mutant RNA polymerase is RNA polymerase derived from T7 phage that has been rendered deficient in at least one of the three amino acid residues 178 through 180.

32. The mutant RNA polymerase of claim 2, wherein the mutant RNA polymerase is RNA polymerase rendered deficient in the 172 lysine and/or 173 arginine amino acid residues of RNA polymerase derived from T7 phage; and rendered deficient in at least one of the three amino acid residues 178 through 180.

33. The mutant RNA polymerase of claim 2, wherein the mutant RNA polymerase is RNA polymerase derived from T3 phage that has been rendered deficient in the 173 lysine and/or 174 arginine amino acid residues of RNA polymerase.

34. The mutant RNA polymerase of claim 2, wherein the mutant RNA polymerase is RNA polymerase derived from T3 phage that has been rendered deficient in at least one of the three amino acid residues 179 through 181.

35. The mutant RNA polymerase of claim 2, wherein the mutant RNA polymerase is RNA polymerase derived from T3 phage that has been rendered deficient in the 173 lysine and/or 174 arginine amino acid residue; and rendered deficient in at least one of the three amino acid residues 179 through 181.

36. The mutant RNA polymerase of claim 2, wherein the mutant RNA polymerase is RNA polymerase derived from K11 phage that has been rendered deficient in the 192 lysine and/or 193 arginine amino acid residues of RNA polymerase.

37. The mutant RNA polymerase of claim 2, wherein the mutant RNA polymerase is RNA polymerase derived from K11 phage that has been rendered deficient in at least one of the three amino acid residues 198 through 200.

38. The mutant RNA polymerase of claim 2, wherein the mutant RNA polymerase is RNA polymerase derived from K11 phage that has been rendered deficient in the 192 lysine and/or 193 arginine amino acid residue; and rendered deficient in at least one of the three amino acid residues 198 through 200.

39. The mutant RNA polymerase of claim 2, wherein the mutant RNA polymerase is RNA polymerase derived from SP6 phage that has been rendered deficient in the 136 lysine and/or 137 arginine amino acid residues of RNA polymerase.

40. The mutant RNA polymerase of claim 2, wherein the mutant RNA polymerase is RNA polymerase derived from SP6 phage that has been rendered deficient in at least one of the four amino acid residues 140 through 143.

41. The mutant RNA polymerase of claim 2, wherein the mutant RNA polymerase is RNA polymerase derived from SP6 phage that has been rendered deficient in the 136 lysine and/or 137 arginine amino acid residue; and rendered deficient in at least one of the four amino acid residues 140 through 143.


SEQUENCE LISTING

<110> NIPPON GENE TECH

<120> Mutant RNA Polymerase

<160> 4

<210> 1

<211> 31

<212> DNA

<213> T7 RNA Polymerase

<400> tacaagaaagcatttatgcaagttgtcgagg

<210> 2

<211> 30

<212> DNA

<213> T7 RNA Polymerase

<400> gacgtgccctacgttgagttgttcctcaac

<210> 3

<211> 31

<212> DNA

<213> T3 RNA Polymerase

<400> tacaagaaagcatttatgcaggtggtcgagg

<210> 4

<211> 30

<212> DNA
<213> T3 RNA Polymerase
<400> gacttgcccgtggttaagctgttcctcaac